(19)

Europäisches Patentamt

European Patent Office

Office européen des brevets

(11) **EP 4 756 019 A2**

(12) **EUROPEAN PATENT APPLICATION**

(43) Date of publication:
**10.06.2026  Bulletin 2026/24**

(21) Application number: **26169348.5**

(22) Date of filing: **30.03.2026**

(51) International Patent Classification (IPC):
*C12N 9/26* (2006.01)    *A61K 38/47* (2006.01)
*G01N 33/68* (2006.01)

(52) Cooperative Patent Classification (CPC):
**C12Y 302/01035; A61K 38/47; C12N 9/2474;
G01N 33/6848**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **19.06.2025  CN 202510827503**

(71) Applicant: **QILU PHARMACEUTICAL CO., LTD.
Jinan, Shandong 250100 (CN)**

(72) Inventors:
• **LI, Hai
Jinan, Shandong, 250100 (CN)**
• **DAI, Huaiqian
Jinan, Shandong, 250100 (CN)**
• **ZHOU, Zuxia
Jinan, Shandong, 250100 (CN)**

• **WANG, Guijiang
Jinan, Shandong, 250100 (CN)**
• **AN, Zhenming
Jinan, Shandong, 250100 (CN)**
• **WANG, Qingmin
Jinan, Shandong, 250100 (CN)**
• **LI, Daoyuan
Jinan, Shandong, 250100 (CN)**
• **LIU, Chuanlei
Jinan, Shandong, 250100 (CN)**
• **WANG, Xiju
Jinan, Shandong, 250100 (CN)**

(74) Representative: **Bandpay & Greuter
11 rue Christophe Colomb
75008 Paris (FR)**

Remarks:
The complete document including Reference
Table(s) and the Sequence Listing(s) can be
downloaded from the EPO website

(54) **A COMPOSITION COMPRISING RECOMBINANT HUMAN HYALURONIDASE AND VARIANTS
THEREOF, AND RELATED METHODS AND USES**

(57)    The present disclosure provides a composition comprising recombinant human hyaluronidase and variants thereof, wherein the variants are truncated variants or oxidatively modified variants of the recombinant human hyaluronidase. Also provided is a pharmaceutical formulation comprising the composition. Further provided is a method for detecting the variants and use of the variants in the quality inspection or quality control of a recombinant human hyaluronidase-containing product.

EP 4 756 019 A2

## Description

### Cross-Reference to Related Application

The present application claims priority to Chinese Patent Application No. 202510827503.4, filed on June 19, 2025, the entire contents of which are incorporated herein by reference for all purposes.

### Technical Field

The present application relates to the field of biomedicines. In particular, the present disclosure provides a composition comprising recombinant human hyaluronidase and variants thereof, as well as related methods and uses.

### Background

Recombinant human hyaluronidase (rHuPH20) has a unique function of degrading hyaluronic acid. Hyaluronic acid is a polysaccharide widely present in human tissues, especially abundant in the skin, synovial fluid and vitreous humor of the eye. Due to its excellent moisturizing and lubricating properties, hyaluronic acid has a wide range of applications in the medical and cosmetic fields. However, in some cases, such as cataract surgery or soft tissue injection, excessive hyaluronic acid may impede drug diffusion and surgical procedures, and therefore, hyaluronidase is required to adjust its concentration.

Clinically, recombinant human hyaluronidase is mainly used to enhance the effects of local anesthesia, promote subcutaneous drug diffusion, assist in ophthalmic surgeries, and treat adverse reactions caused by hyaluronic acid injections, and the like. Its use has greatly improved surgical safety and efficiency and improved the treatment experience for patients. Traditional hyaluronidase is extracted from animal testes, which is not only costly, but also may elicit immune responses. The emergence of recombinant human hyaluronidase addresses these problems.

As a drug, recombinant human hyaluronidase is required to maintain necessary activity and efficacy. The quality control for the drug mainly involves controlling the content of the active ingredient and other related substances such as variants or impurities, and in particular, the content of these substances needs to meet pharmaceutical requirements. Analysis and detection of variants or impurities of recombinant human hyaluronidase are of great significance for the research and development of effective products and formulations comprising recombinant human hyaluronidase.

### Summary of the Invention

In a first aspect, the present disclosure provides a composition comprising recombinant human hyaluronidase and a variant thereof, wherein the variant is a truncated variant resulting from a cleavage between positions $R^{311}$ and $S^{312}$ of the recombinant human hyaluronidase, wherein the content of the truncated variant is less than or equal to about 10% of the total amount of the recombinant human hyaluronidase and the variant thereof in the composition, calculated based on a Lys-C digestion peptide mass fingerprinting analysis; and wherein the positions are numbered with reference to the sequence as set forth in any one of SEQ ID NOs: 1-4, for example the sequence as set forth in SEQ ID NO: 1.

In particular embodiments, the content of the truncated variant is less than or equal to about 10% of the total amount of the recombinant human hyaluronidase and the variant thereof in the composition, calculated based on the Lys-C digestion peptide mass fingerprinting analysis.

In some embodiments, the content of the recombinant human hyaluronidase is greater than or equal to about 90% of the total amount of the recombinant human hyaluronidase and the variant thereof in the composition, calculated based on the Lys-C digestion peptide mass fingerprinting analysis.

In some embodiments, the content of the truncated variant is greater than or equal to about 0.01%, greater than or equal to about 0.05%, greater than or equal to about 0.1%, greater than or equal to about 0.5%, greater than or equal to about 1%, greater than or equal to about 1.5%, greater than or equal to about 2%, greater than or equal to about 2.5%, or greater than or equal to about 3% of the total amount of the recombinant human hyaluronidase and the variant thereof in the composition, calculated based on the Lys-C digestion peptide mass fingerprinting analysis.

In some embodiments, the content of the truncated variant is less than or equal to about 9%, less than or equal to about 8%, less than or equal to about 7%, less than or equal to about 6%, less than or equal to about 5%, less than or equal to about 4.5%, less than or equal to about 4%, less than or equal to about 3%, less than or equal to about 2%, less than or equal to about 1%, less than or equal to about 0.9%, or less than or equal to about 0.8%, less than or equal to about 0.7%, less than or equal to about 0.6%, less than or equal to about 0.5%, less than or equal to about 0.4%, less than or equal to about 0.3%, less than or equal to about 0.2%, or less than or equal to about 0.1% of the total amount of the recombinant human hyaluronidase and the variant thereof in the composition, calculated based on the Lys-C digestion peptide mass fingerprinting analysis.

In some embodiments, the content of the truncated variant can be any interval within the ranges defined by any two of the above-mentioned values or any value within the intervals.

In some embodiments, the composition further comprises another variant that is an oxidatively modified variant resulting from oxidation at position $M^{310}$ and an oxidatively modified variant resulting from oxidation at position $M^{313}$ of recombinant human hyaluronidase, wherein the sum of the contents of the oxidatively modified variants is less than or equal to about 15% of the total amount of the recombinant human hyaluronidase and variants thereof in the composition, calculated based on the Lys-C digestion peptide mass fingerprinting analysis; and wherein the positions are numbered with reference to the sequence as set forth in any one of SEQ ID NOs:1-4, for example, the sequence as set forth in SEQ ID NO:1.

Preferably, the sum of the contents of the oxidatively modified variants is greater than or equal to about 0.01%, greater than or equal to about 0.05%, greater than or equal to about 0.1%, greater than or equal to about 0.5%, greater than or equal to about 1%, greater than or equal to about 1.5%, greater than or equal to about 2%, greater than or equal to about 2.5%, greater than or equal to about 3%, greater than or equal to about 3.5%, greater than or equal to about 4%, greater than or equal to about 4.5%, or greater than or equal to about 5% of the total amount of the recombinant human hyaluronidase and variants thereof in the composition, calculated based on the Lys-C digestion peptide mass fingerprinting analysis.

In some embodiments, the recombinant human hyaluronidase comprises an amino acid sequence as set forth in any one of SEQ ID NOs: 1-4. In some embodiments, the full-length sequence of the recombinant human hyaluronidase is as set forth in any one of SEQ ID NOs: 1-4. In some embodiments, the recombinant human hyaluronidase is produced by N-terminal or C-terminal truncation and/or amino acid residue modification (including substitution, deletion, and insertion) based on the sequence as set forth in SEQ ID NO: 1.

In some specific embodiments, the sequence of the truncated variant is as set forth in SEQ ID NO: 5.

In some embodiments, the steps of calculating the content of the truncated variant based on Lys-C digestion peptide mass fingerprinting analysis comprises: digesting the composition with Lys-C followed by the peptide mass fingerprinting analysis by using LC-MS/MS; extracting the extracted ion chromatograms (XIC) of two charge forms with the strongest responses of the truncated peptide segment and its corresponding native peptide segment after obtaining mass spectrometric data by LC-MS/MS analysis; integrating to obtain respective peak area; and calculating the proportion of the truncated variant according to the following formula:

The proportion of truncated modified variant = (XIC area of truncated modified peptide segment)/(XIC area of truncated modified peptide segment+XIC area of native peptide segment) $\times$ 100%.

In some specific embodiments, the XIC area of the truncated peptide segment is the peak area obtained by integrating the XIC chromatogram of the truncated modified peptide segment (hereinafter referred to as the "truncated peptide segment", the sequence of which is as set forth in SEQ ID NO:7) extracted from the mass spectrometric data of the sample, and the XIC area of the native peptide segment is the peak area obtained by integrating the XIC chromatogram of the peptide segment without modification by truncation (hereinafter referred to as the "native peptide segment", the sequence of which is as set forth in SEQ ID NO:6).

In some embodiments, the steps of calculating the content of the oxidized variants based on Lys-C digestion peptide mass fingerprinting analysis comprises : extracting the extracted ion chromatograms (XIC) of two charge forms with the strongest responses of the oxidatively modified peptide segments and their corresponding native peptide segments after obtaining mass spectrometric data by LC-MS/MS analysis; integrating to obtain respective peak area; and calculating the content of the oxidatively modified variants according to the following formula:

The proportion of oxidatively modified variants = (XIC area of oxidatively modified peptide segments)/(XIC area of oxidatively modified peptide segments + XIC area of native peptide segment)$\times$100% .

In some specific embodiments, the oxidatively modified peptide segment is a peptide segment as set forth in SEQ ID NO:8 or SEQ ID NO:9. In specific embodiments, the XIC area of the oxidatively modified peptide segments is the peak area corresponding to the peptide segment as set forth in SEQ ID NO:8 plus the peak area corresponding to the peptide segment as set forth in SEQ ID NO:9, and the XIC area of the native peptide segment is the peak area obtained by integrating the XIC of the peptide segment without modification (hereinafter referred to as the "native peptide segment", the sequence of which is as set forth in SEQ ID NO:6).

In a second aspect, the present disclosure provides a composition comprising recombinant human hyaluronidase and a variant thereof, wherein the variant is an oxidatively modified variant resulting from oxidation at position $M^{310}$ and an oxidatively modified variant resulting from oxidation at position $M^{313}$ of the recombinant human hyaluronidase, wherein the sum of the contents of the oxidatively modified variants is less than or equal to about 15% of the total amount of the recombinant human hyaluronidase and variants thereof in the composition, calculated based on a Lys-C digestion peptide mass fingerprinting analysis; and wherein the positions are numbered with reference to the sequence as set forth in any one of SEQ ID NOs: 1-4, for example the sequence as set forth in SEQ ID NO:1.

In particular embodiments, the sum of the oxidatively modified variants is less than or equal to about 15% of the total amount of the recombinant human hyaluronidase and variants thereof, calculated based on the Lys-C digestion peptide mass fingerprinting analysis.

In some embodiments, the content of the recombinant human hyaluronidase in the composition is greater than or equal to about 85% of the total amount of the recombinant human hyaluronidase and variants thereof, for example, calculated based on the Lys-C digestion peptide mass fingerprinting analysis.

In some embodiments, the sum of the contents of the oxidatively modified variants is greater than or equal to about 0.01%, greater than or equal to about 0.05%, greater than or equal to about 0.1%, greater than or equal to about 0.5%, greater than or equal to about 1%, greater than or equal to about 1.5%, greater than or equal to about 2%, greater than or equal to about 2.5%, or greater than or equal to about 3%, greater than or equal to about 3.5%, greater than or equal to about 4%, greater than or equal to about 4.5%, or greater than or equal to about 5% of the total amount of the recombinant human hyaluronidase and variants thereof, calculated based on the Lys-C digestion peptide mass fingerprinting analysis.

In some embodiments, the sum of the contents of the oxidatively modified variants is less than or equal to about 14%, less than or equal to about 13%, less than or equal to about 12%, less than or equal to about 11%, less than or equal to about 10%, less than or equal to about 9%, less than or equal to about 8%, less than or equal to about 7%, less than or equal to about 6%, less than or equal to about 5%, less than or equal to about 4%, less than or equal to about 3%, less than or equal to about 2%, less than or equal to about 1%, less than or equal to about 0.9%, less than or equal to about 0.8%, less than or equal to about 0.7%, less than or equal to about 0.6%, less than or equal to about 0.5%, less than or equal to about 0.4%, less than or equal to about 0.3%, less than or equal to about 0.2%, or less than or equal to about 0.1% of the total amount of the recombinant human hyaluronidase and variants thereof, calculated based on the Lys-C digestion peptide mass fingerprinting analysis.

In some embodiments, the content of the oxidatively modified variants can be any interval within the ranges defined by any two of the above-mentioned values or any value within the intervals.

In some embodiments, the composition further comprises another variant that is a truncated variant resulting from a cleavage between positions $R^{311}$ and $S^{312}$ of the recombinant human hyaluronidase, wherein the content of the truncated variant is less than or equal to about 10% of the total amount of the recombinant human hyaluronidase and variants thereof in the composition, calculated based on the Lys-C digestion peptide mass fingerprinting analysis; and wherein the positions are numbered with reference to the sequence as set forth in any one of SEQ ID NOs:1-4. Preferably, the content of the truncated variant is greater than or equal to about 0.01%, greater than or equal to about 0.05%, greater than or equal to about 0.1%, greater than or equal to about 0.5%, greater than or equal to about 1%, greater than or equal to about 1.5%, greater than or equal to about 2%, greater than or equal to about 2.5%, or greater than or equal to about 3% of the total amount of the recombinant human hyaluronidase and variants thereof in the composition, calculated based on the Lys-C digestion peptide mass fingerprinting analysis.

In some embodiments, the recombinant human hyaluronidase comprises an amino acid sequence as set forth in any one of SEQ ID NOs: 1-4. In some embodiments, the full-length sequence of the recombinant human hyaluronidase is as set forth in any one of SEQ ID NOs: 1-4. In some embodiments, the recombinant human hyaluronidase is produced by N-terminal or C-terminal truncation and/or amino acid residue modification (including substitution, deletion, and insertion) based on the sequence as set forth in SEQ ID NO:1.

In some embodiments, the oxidatively modified variant is produced by the oxidation of methionine (M) at position 310 of the sequence as set forth in any one of SEQ ID NOs: 1-4.

In some embodiments, the oxidatively modified variant is produced by the oxidation of methionine (M) at position 313 of the sequence as set forth in any one of SEQ ID NOs: 1-4.

In some embodiments, the steps of calculating the contents of the oxidized variants based on the Lys-C digestion peptide mass fingerprinting analysis comprise: extracting the extracted ion chromatograms (XICs) of two charge forms which have the strongest responses of the oxidatively modified peptide segments and their corresponding native peptide segments after obtaining mass spectrometric data by LC-MS/MS analysis; integrating to obtain respective peak area; and calculating the content of the oxidatively modified variants according to the following formula:

The proportion of oxidatively modified variants = (XIC area of oxidatively modified peptide segments)/(XIC area of oxidatively modified peptide segments + XIC area of native peptide segment) $\times$ 100%.

In some specific embodiments, the oxidatively modified peptide segment is a peptide segment as set forth in SEQ ID NO:8 or SEQ ID NO:9. In specific embodiments, the XIC area of the oxidatively modified peptide segments is the peak area corresponding to the peptide segment as set forth in SEQ ID NO:8 plus the peak area corresponding to the peptide segment as set forth in SEQ ID NO:9, and XIC area of the native peptide segment is the peak area obtained by integrating the XIC of the peptide segment without modification (hereinafter referred to as the "native peptide segment", the sequence of which is as set forth in SEQ ID NO:6).

In some embodiments, the steps of calculating the content of the truncated variant based on Lys-C digestion peptide mass fingerprinting analysis comprises : digesting the composition with Lys-C followed by the peptide mass fingerprinting analysis by using LC-MS/MS; extracting the extracted ion chromatograms (XIC) of two charge forms which have the strongest responses of the truncated peptide segment and its corresponding native peptide segment after obtaining mass spectrometric data by LC-MS/MS analysis; integrating to obtain respective peak area; and calculating the proportion of the truncated variant according to the following formula:

The proportion of truncated modified variant = (XIC area of truncated modified peptide segment)/(XIC area of truncated modified peptide segment + XIC area of native peptide segment) $\times$ 100%.

In some specific embodiments, the XIC area of the truncated peptide segment is the peak area obtained by integrating the XIC of the truncated modified peptide segment (hereinafter referred to as the "truncated peptide segment", the sequence of which is as set forth in SEQ ID NO:7) extracted from the mass spectrometric data of the sample, and the XIC area of the native peptide segment is the peak area obtained by integrating the XIC of the peptide segment without modification by truncation (hereinafter referred to as the "native peptide segment", the sequence of which is as set forth in SEQ ID NO:6).

In a third aspect, the present disclosure provides a pharmaceutical formulation comprising the composition of the first aspect and/or the second aspect, and one or more pharmaceutically acceptable carriers. In some embodiments, the pharmaceutical formulation further comprises an additional pharmaceutically active ingredient; preferably, the pharmaceutically active ingredient is selected from the group consisting of immunoglobulins, recombinant proteins, synthetic polypeptides, RNA, DNA and chemical drugs.

In a fourth aspect, the present disclosure provides a method for preparing the pharmaceutical formulation of the third aspect, comprising the steps of:

(1) preparing a composition comprising recombinant human hyaluronidase and variants thereof, wherein the variants are the truncated variants as defined in the first aspect or the oxidatively modified variants as defined in the second aspect above, and
(2) assessing the variants in the composition and confirming that the content of the variants does not significantly affect the activity of the recombinant human hyaluronidase.

For example, the content of the truncated variant should be less than or equal to about 10% of the total amount of the recombinant human hyaluronidase and variants thereof in the composition, and the content of the oxidatively modified variants should be less than or equal to about 15% of the total amount of the recombinant human hyaluronidase and variants thereof in the composition.

In some embodiments, the above step (2) comprises subjecting the composition to peptide mass fingerprinting analysis to obtain XIC chromatograms of variant peptide segments and native peptide segments, and calculating the contents of the variants from the corresponding peak areas.

In some embodiments, the method further comprises combining the composition obtained after the above step (2) with a pharmaceutically acceptable carrier.

In a fifth aspect, the present disclosure provides a method for detecting a recombinant human hyaluronidase variant in a composition or pharmaceutical formulation comprising recombinant human hyaluronidase, wherein the variant is a truncated variant resulting from a cleavage between positions $R^{311}$ and $S^{312}$ of the recombinant human hyaluronidase, and wherein the positions are numbered with reference to the sequence as set forth in any one of SEQ ID NOs:1-4, the method comprising the following steps: digesting the composition or pharmaceutical formulation followed by a peptide mass fingerprinting analysis, for example, by using LC-MS/MS to obtain XIC chromatograms of the truncated peptide segment and the native peptide segment, and calculating the content of the truncated variant from the corresponding peak areas.

In a sixth aspect, the present disclosure provides a method for detecting a recombinant human hyaluronidase variant in a composition or pharmaceutical formulation comprising recombinant human hyaluronidase, wherein the variant is an oxidatively modified variant resulting from oxidation at position $M^{310}$ and an oxidatively modified variant resulting from oxidation at position $M^{313}$ of the recombinant human hyaluronidase, and wherein the positions are numbered with reference to the sequence as set forth in any one of SEQ ID NOs:1-4, the method comprising the following steps: digesting the composition or pharmaceutical formulation followed by a peptide mass fingerprinting analysis, for example, by using LC-MS/MS to obtain XIC chromatograms of the peptide segments of the oxidatively modified variants and the native peptide segment, and calculating the content of the oxidatively modified variants from the corresponding peak areas.

In a seventh aspect, the present disclosure provides a method for quality inspection or quality control of a recombinant human hyaluronidase-containing product, comprising detecting the content of a recombinant human hyaluronidase variant in the product. In some embodiments, the variant is a truncated variant resulting from a cleavage between positions $R^{311}$ and $S^{312}$ of the recombinant human hyaluronidase. In some embodiments, the variant is an oxidatively modified variant resulting from oxidation at position $M^{310}$ and an oxidatively modified variant resulting from oxidation at position $M^{313}$ of the recombinant human hyaluronidase. The positions are numbered with reference to the sequence as set forth in any one of SEQ ID NOs: 1-4.

In some embodiments, the recombinant human hyaluronidase-containing product is the composition of the first aspect or the second aspect or the pharmaceutical formulation of the third aspect.

In some embodiments, the recombinant human hyaluronidase meets medicinal requirements if the content of the truncated variant is detected to be less than or equal to about 10% of the total amount of the recombinant human hyaluronidase and variants thereof in the composition.

In some embodiments, the recombinant human hyaluronidase meets medicinal requirements if the content of the oxidatively modified variants is detected to be less than or equal to about 15% of the total amount of the recombinant human hyaluronidase and variants thereof in the composition.

In an eighth aspect, the present disclosure provides use of a recombinant human hyaluronidase variant in the quality inspection or quality control of a recombinant human hyaluronidase-containing product. In some embodiments, the variant is a truncated variant resulting from a cleavage between positions $R^{311}$ and $S^{312}$ of the recombinant human hyaluronidase. In some embodiments, the variant is an oxidatively modified variant resulting from oxidation at position $M^{310}$ and an oxidatively modified variant resulting from oxidation at position $M^{313}$ of the recombinant human hyaluronidase. The positions are numbered with reference to the sequence as set forth in any one of SEQ ID NOs: 1-4.

In some embodiments, the recombinant human hyaluronidase-containing product is the composition of the first aspect or the second aspect or the pharmaceutical formulation of the third aspect.

## Brief Description of the Drawings

Figure 1 shows the deconvoluted spectrum result of non-reduced intact molecular weight analysis of the recombinant human hyaluronidase after digesting N-glycan. Note: "Dea" denotes deamidation modification; "-Y" denotes C-terminal truncation of tyrosine (Y); "$R^{311}/S^{312}$" denotes that a cleavage modification between $R^{311}$ and $S^{312}$; and the numbers in parentheses indicate the number of modifications that occur in the component.

Figure 2 shows the results of the MS1 spectra and MS2 spectra of the $F^{280}$-$R^{311}$ peptide segment detected after digesting the recombinant human hyaluronidase sample with Lys-C.

Figure 3 shows the standard curve obtained by plotting recombinant human hyaluronidase samples with different activities against their corresponding average absorbance. The standard curve equation is as follows: Y=0.412-0.008x, R2=0.989.

Figure 4 shows a typical chromatogram of the components collected by RP-HPLC, which is obtained by synchronously collecting the main peak and the impurity peak in front of the main peak of the recombinant human hyaluronidase sample using the Agilent 1260 liquid chromatography automatic fraction collector module.

Figure 5 shows the deconvoluted spectrum results of N-glycan cleaved non-reduced intact molecular weight analysis of the impurity peak separated and collected by RP-HPLC from recombinant human hyaluronidase sample and the stock solution sample.

Figure 6 shows the MS2 spectra analysis results of the $M^{310}$ and $M^{313}$ oxidatively modified peptide segments in the impurity peak sample separated and collected by RP-HPLC.

Figure 7 shows a chromatogram for confirming localization of the collected components by RP-HPLC.

Figure 8 shows a typical RP-HPLC chromatogram of the light-exposed samples and the control sample.

## Description of the Sequences

SEQ ID NO:1 is the amino acid sequence of human hyaluronidase with 447 amino acid residues:

LNFRAPPVIPNVPFLWAWNAPSEFCLGKFDEPLDMSLFSFIGSPRINATGQG

VTIFYVDRLGYYPYIDSITGVTVNGGIPQKISLQDHLDKAKKDITFYMPVDNL

GMAVIDWEEWRPTWARNWKPKDVYKNRSIELVQQQNVQLSLTEATEKAKQE

FEKAGKDFLVETIKLGKLLRPNHLWGYYLFPDCYNHHYKKPGYNGSCFNVEI

KRNDDLSWLWNESTALYPSIYLNTQQSPVAATLYVRNRVREAIRVSKIPDAKSP

LPVFAYTRIVFTDQVLKFLSQDELVYTFGETVALGASGIVIWGTLSIMRSMKSC

LLLDNYMETILNPYIINVTLAAKMCSQVLCQEQGVCIRKNWNSSDYLHLNPD

NFAIQLEKGGKFTVRGKPTLEDLEQFSEKFYCSCYSTLSCKEKADVKDTDAVD

VCIADGVCIDAFLKPPMETEEPQIFY.

SEQ ID NO:2 is the amino acid sequence of human hyaluronidase with one amino acid (i.e., tyrosine (Y)) deleted at the C-terminal of the polypeptide sequence as set forth in SEQ ID NO:1:

LNFRAPPVIPNVPFLWAWNAPSEFCLGKFDEPLDMSLFSFIGSPRINATGQG

VTIFYVDRLGYYPYIDSITGVTVNGGIPQKISLQDHLDKAKKDITFYMPVDNL

GMAVIDWEEWRPTWARNWKPKDVYKNRSIELVQQQNVQLSLTEATEKAKQE

FEKAGKDFLVETIKLGKLLRPNHLWGYYLFPDCYNHHYKKPGYNGSCFNVEI

KRNDDLSWLWNESTALYPSIYLNTQQSPVAATLYVRNRVREAIRVSKIPDAKSP

LPVFAYTRIVFTDQVLKFLSQDELVYTFGETVALGASGIVIWGTLSIMRSMKSC

LLLDNYMETILNPYIINVTLAAKMCSQVLCQEQGVCIRKNWNSSDYLHLNPD

NFAIQLEKGGKFTVRGKPTLEDLEQFSEKFYCSCYSTLSCKEKADVKDTDAVD

VCIADGVCIDAFLKPPMETEEPQIF.

SEQ ID NO:3 is the amino acid sequence of human hyaluronidase with two amino acids (i.e., tyrosine (Y) and phenylalanine (F)) deleted at the C-terminal of the polypeptide sequence as set forth in SEQ ID NO:1:

LNFRAPPVIPNVPFLWAWNAPSEFCLGKFDEPLDMSLFSFIGSPRINATGQG

VTIFYVDRLGYYPYIDSITGVTVNGGIPQKISLQDHLDKAKKDITFYMPVDNL

GMAVIDWEEWRPTWARNWKPKDVYKNRSIELVQQQNVQLSLTEATEKAKQE

FEKAGKDFLVETIKLGKLLRPNHLWGYYLFPDCYNHHYKKPGYNGSCFNVEI

KRNDDLSWLWNESTALYPSIYLNTQQSPVAATLYVRNRVREAIRVSKIPDAKSP

LPVFAYTRIVFTDQVLKFLSQDELVYTFGETVALGASGIVIWGTLSIMRSMKSC

LLLDNYMETILNPYIINVTLAAKMCSQVLCQEQGVCIRKNWNSSDYLHLNPD

NFAIQLEKGGKFTVRGKPTLEDLEQFSEKFYCSCYSTLSCKEKADVKDTDAVD

VCIADGVCIDAFLKPPMETEEPQI.

SEQ ID NO:4 is the amino acid sequence of human hyaluronidase with three amino acids (i.e., tyrosine (Y), phenylalanine (F) and isoleucine (I)) deleted at the C-terminal of the polypeptide sequence as set forth in SEQ ID NO:1:

LNFRAPPVIPNVPFLWAWNAPSEFCLGKFDEPLDMSLFSFIGSPRINATGQG
VTIFYVDRLGYYPYIDSITGVTVNGGIPQKISLQDHLDKAKKDITFYMPVDNL
GMAVIDWEEWRPTWARNWKPKDVYKNRSIELVQQQNVQLSLTEATEKAKQE
FEKAGKDFLVETIKLGKLLRPNHLWGYYLFPDCYNHHYKKPGYNGSCFNVEI
KRNDDLSWLWNESTALYPSIYLNTQQSPVAATLYVRNRVREAIRVSKIPDAKSP
LPVFAYTRIVFTDQVLKFLSQDELVYTFGETVALGASGIVIWGTLSIMRSMKSC
LLLDNYMETILNPYIINVTLAAKMCSQVLCQEQGVCIRKNWNSSDYLHLNPD
NFAIQLEKGGKFTVRGKPTLEDLEQFSEKFYCSCYSTLSCKEKADVKDTDAVD
VCIADGVCIDAFLKPPMETEEPQ.

SEQ ID NO:5 is an exemplary truncated variant sequence resulting from a cleavage between amino acid residues $R^{311}$ and $S^{312}$ of the above sequence as set forth in SEQ ID NO: 4. The cleavage between amino acid residues $R^{311}$ and $S^{312}$ can occur in all polypeptide sequences including but not limited to the polypeptide sequences as set forth in SEQ ID NOs: 1-4 and those resulting from N-terminal or C-terminal truncation and/or amino acid residue modification (including substitution, deletion and insertion). After the cleavage between $R^{311}$ and $S^{312}$, the molecules can still be linked together by $C^{25}=C^{316}$ disulfide bond. Here, the sequence as set forth in SEQ ID NO: 4 exemplarily shows the structure of the truncated (the cleavage site is marked with "/") variant and the linkage of $C^{25}=C^{316}$ disulfide bond (underlined in the sequence):

LNFRAPPVIPNVPFLWAWNAPSEFCLGKFDEPLDMSLFSFIGSPRINATG
QGVTIFYVDRLGYYPYIDSITGVTVNGGIPQKISLQDHLDKAKKDITFYMPV
DNLGMAVIDWEEWRPTWARNWKPKDVYKNRSIELVQQQNVQLSLTEATEK
AKQEFEKAGKDFLVETIKLGKLLRPNHLWGYYLFPDCYNHHYKKPGYNGS
CFNVEIKRNDDLSWLWNESTALYPSIYLNTQQSPVAATLYVRNRVREAIRVS
KIPDAKSPLPVFAYTRIVFTDQVLKFLSQDELVYTFGETVALGASGIVIWGTL
SIMR³¹¹/SMKSCLLLDNYMETILNPYIINVTLAAKMCSQVLCQEQGVCIRKN
WNSSDYLHLNPDNFAIQLEKGGKFTVRGKPTLEDLEQFSEKFYCSCYSTLSC
KEKADVKDTDAVDVCIADGVCIDAFLKPPMETEEPQ↵

SEQ ID NO:6 is the sequence of the native peptide segment used for calculating the content of the variants in Examples 4 and 5, which corresponds to the amino acids at positions 280-314 of any of the sequences as set forth in SEQ ID NOs:1-4:
FLSQDELVYTFGETVALGASGIVIWGTLSIM$R^{311}S^{312}$MK.
SEQ ID NO:7 is the sequence of the truncated peptide segment used for calculating the content of the truncated variant in Example 4, which corresponds to the amino acids at positions 280-311 of any of the sequences as set forth in SEQ ID NOs:1-4:
FLSQDELVYTFGETVALGASGIVIWGTLSIM$R^{311}$
SEQ ID NO:8 is the sequence of the oxidatively modified peptide segment used for calculating the contents of the oxidatively modified variants in Example 5, which corresponds to the amino acids at positions 280-314 of any of the sequences as set forth in SEQ ID NOs:1-4, wherein methionine at position 310 undergoes oxidation (Oxi):
FLSQDELVYTFGETVALGASGIVIWGTLSI$M^{310}(Oxi)$
SEQ ID NO:9 is the sequence of the oxidatively modified peptide segment used for calculating the contents of the oxidatively modified variants in Example 5, which corresponds to the amino acids at positions 280-314 of any of the sequences as set forth in SEQ ID NOs:1-4, wherein methionine at position 313 undergoes oxidation (Oxi):

FLSQDELUYTFGETVALGASGIVIWGTLSIMRS$\underline{M^{313}}$(Oxi)

## Detailed Description

Hyaluronidase is a general term for enzymes capable of hydrolyzing hyaluronic acid, and is an enzyme capable of reducing the activity of hyaluronic acid *in vivo* so as to improve liquid permeability in tissues. Many pathological changes are often accompanied by changes in hyaluronidase and hyaluronic acid, which can alter the distribution of some drugs and physiologically active substances in the body.

As used herein, "recombinant human hyaluronidase" refers to human hyaluronidase (also known as hyaluronidase) expressed by genetic engineering means. Exemplary recombinant human hyaluronidase can be selected from the rHuPH20 polypeptides as set forth in any one of SEQ ID NOs:1-4. Hyaluronidase may also include those with chemical or post-translational modifications and those without chemical or post-translational modifications. Such modifications include, but are not limited to, pegylation, albumination, glycosylation, famesylation, carboxylation, hydroxylation, phosphorylation, and other polypeptide modifications known in the art.

Both hyaluronidase and recombinant human hyaluronidase have the function of degrading hyaluronic acid, and are widely used in the medical and cosmetic fields. The recombinant human hyaluronidase is produced by genetic engineering technology, which has higher safety and production efficiency, and avoids the limitations of traditional extraction methods. The recombinant human hyaluronidase is produced by inserting the gene encoding for human hyaluronidase into an appropriate expression vector followed by expression in host cells, such as mammal cells or bacteria. It has been found that during the recombinant expression of human hyaluronidase, the C-terminal deletion of 1-3 amino acids may occur, and such deletion does not affect the enzyme activity. For example, SEQ ID NO:2 lacks tyrosine (Y) at the C-terminus of SEQ ID NO:1, SEQ ID NO:3 lacks tyrosine (Y) and phenylalanine (F) at the C-terminus of SEQ ID NO:1, and SEQ ID NO:4 lacks tyrosine (Y), phenylalanine (F) and isoleucine (I) at the C-terminus of SEQ ID NO:1. In addition to the recombinant human hyaluronidase as set forth in SEQ ID NO:1, the sequences as set forth in SEQ ID NO:2-4 are also common expression forms of the recombinant human hyaluronidase. The above several forms can coexist in the recombinant human hyaluronidase samples or formulations. In addition, a variety of studies have been carried out in the prior art on the basis of the polypeptide sequence of the recombinant human hyaluronidase as set forth in SEQ ID NO: 1, such as truncation of the N-terminus and/or C-terminus and modification of amino acid residues (including substitution, deletion and insertion). For example, truncating to positions 1-432 of the sequence shown in SEQ ID NO:1 can still retain the hyaluronidase activity (see, for example, Chinese Patent Nos. CN104244968B and CN112203642B, and Gregory I Frost; Recombinant human hyaluronidase (rHuPH20): an enabling platform for subcutaneous drug and fluid administration; Expert Opinion on Drug Delivery, 2007, 4 (4), 427-440). Therefore, "recombinant human hyaluronidase" as used herein includes rHuPH20 polypeptides having the amino acid sequence as set forth in any one of SEQ ID NOs:1-4, those with N-terminal or C-terminal truncation on the basis of the amino acid sequence as set forth in SEQ ID NO:1, or those with amino acid residue modifications (including substitution, deletion, and insertion) on the basis of the amino acid sequence as set forth in SEQ ID NO:1.

The inventors of the present application have unexpectedly found that other variants of human hyaluronidase, such as truncated variants or oxidatively modified variants, have also been produced during the recombinant expression of human hyaluronidase.

In some embodiments, the variant is a truncated variant resulting from the cleavage between amino acid residues $R^{311}$ and $S^{312}$ of the recombinant human hyaluronidase corresponding to the amino acid sequence as set forth in any one of SEQ IDs NO:1-4. After cleavage between amino acid residues $R^{311}$ and $S^{312}$, the polypeptide fragments on both sides of the cleavage site may still be linked together by a disulfide bond ($C^{25}=C^{316}$) between amino acid residues $C^{25}$ and $C^{316}$ corresponding to any of the amino acid sequences as set forth in SEQ ID NOs:1-4. The amino acid sequence of one exemplary truncated variant of the recombinant human hyaluronidase is as set forth in SEQ ID NO:5. The inventors have also found that the activity of the recombinant human hyaluronidase sample will not be significantly affected if the content of the truncated variant is less than or equal to about 10% of the total amount of the recombinant human hyaluronidase and variants thereof. Therefore, if the content of the variant is evaluated or detected to be not higher than about 10% in the process of preparing the recombinant human hyaluronidase, there is no need to perform operations of removing the variant or impurity, which simplifies the production process to some extent and saves the production cost.

In some embodiments, the variant is an oxidatively modified variant resulting from oxidation at position $M^{310}$ and an oxidatively modified variant resulting from oxidation at position $M^{313}$ of the recombinant human hyaluronidase corresponding to the sequence as set forth in any one of SEQ ID NOs: 1-4.

In some specific embodiments, the oxidatively modified variant is produced by the oxidation of methionine (M) at position 310 corresponding to the sequence as set forth in any one of SEQ ID NOs: 1-4. In some specific embodiments, the oxidatively modified variant is produced by the oxidation of methionine (M) at position 313 corresponding to the sequence as set forth in any one of SEQ ID NOs: 1-4. In some specific embodiments, the oxidatively modified variant is an oxidatively modified variant resulting from oxidation at position 310 and an oxidatively modified variant resulting from oxidation of methionine (M) at position 313 corresponding to the sequence as set forth in any one of SEQ ID NOs: 1-4. The side chain structure of methionine is -CH$_2$-CH$_2$-S-CH$_3$, in which the sulfur atom (S) is oxidized to generate sulfoxide, and thus the side chain structure of methionine becomes -CH$_2$-CH$_2$-SO-CH$_3$. Oxidation of methionine will result in changes in the secondary, tertiary or quaternary structure of the protein, thereby affecting its function. For example, the activity of recombinant human hyaluronic acid may be reduced or lost due to oxidation.

The inventors have also found that the activity of the recombinant human hyaluronidase sample will not be significantly affected if the content of the above oxidatively modified variant is less than or equal to about 15% of the total amount of the recombinant human hyaluronidase and variants thereof. Therefore, if the content of the oxidatively modified variant is evaluated or detected to be not higher than about 15% in the process of preparing the recombinant human hyaluronidase, there is no need to perform operations of removing the variant or impurity, which simplifies the production process to some extent and saves the production cost.

In some embodiments, the composition described herein comprises recombinant human hyaluronidase and truncated variants thereof (e.g., as set forth in SEQ ID NO:5).

In some embodiments, the composition described herein comprises recombinant human hyaluronidase and oxidatively modified variants thereof (e.g., an oxidatively modified variant resulting from oxidation at position M$^{310}$ and an oxidatively modified variant resulting from oxidation at position M$^{313}$ corresponding to the sequence as set forth in any one of SEQ ID NOs: 1-4).

In some embodiments, the above truncated variants or oxidatively modified variants in the recombinant human hyaluronidase-containing product can be reduced or removed by methods commonly used in the art such as ion exchange chromatography, hydrophobic chromatography, complex chromatography, or reverse phase HPLC.

In some embodiments, a recombinant human hyaluronidase-containing product, such as a composition or a pharmaceutical preparation comprising recombinant human hyaluronidase, is digested followed by peptide mass fingerprinting analysis to detect variants in the recombinant human hyaluronidase-containing product.

In specific embodiments, the above digestion is carried out using Lys-C. Lys-C (Lysyl endopeptidase) is an endoprotease that can specifically cleave the carboxyl side of Lys (lysine) residues in proteins. It is commonly used for digestion analysis of proteins to study the structure and function of proteins.

In some embodiments, liquid chromatography-tandem mass spectrometry (LC-MS/MS) analysis is used to detect variants in the recombinant human hyaluronidase-containing product. For example, the peptide mass fingerprinting analysis is performed on the test samples using LC-MS/MS. LC-MS/MS (Liquid Chromatography-Tandem Mass Spectrometry) is an analytical technique with high sensitivity and selectivity, which combines the physical separation capability of liquid chromatography (LC) with the accurate detection capability of tandem mass spectrometry (MS/MS). Components in complex samples can be efficiently separated by chromatographic columns, and high-resolution mass spectrometry can analyze the components in the product by mass spectrometry to obtain component composition, confirmation of modification sites, and identification of modification types and so on. This technique combines the high separation ability of liquid chromatography (LC) with the high sensitivity and specificity of mass spectrometry (MS), and is a core tool for drug analysis, metabolic research and quality control.

In specific embodiments, the extracted ion chromatograms (XICs) of two charge forms with the strongest responses of the truncated peptide segment and its corresponding native peptide segment are extracted after obtaining mass spectrometric data by LC-MS/MS analysis; respective peak area is obtained by integration; and the content of the truncated variant is calculated according to the following formula:

The proportion of truncated modified variant = (XIC area of truncated modified peptide segment)/(XIC area of truncated modified peptide segment + XIC area of native peptide segment) $\times$ 100%.

In some embodiments, the truncated peptide segment is a peptide segment as set forth in SEQ ID NO:7 and the native peptide segment is a peptide segment as set forth in SEQ ID NO:6.

In specific embodiments, the extracted ion chromatograms (XICs) of two charge forms with the strongest responses of the oxidatively modified peptide segment and its corresponding native peptide segment are extracted after obtaining mass spectrometric data by LC-MS/MS analysis; respective peak area is obtained by integration; and the content of the oxidatively modified variants is calculated according to the following formula:

The proportion of oxidatively modified variants = (XIC area of oxidatively modified peptide segments)/(XIC area of oxidatively modified peptide segments + XIC area of native peptide segment) $\times$ 100%.

In some embodiments, the oxidatively modified peptide segment is a peptide segment as set forth in SEQ ID NO:8 or SEQ ID NO:9. In specific embodiments, the XIC area of the oxidatively modified peptide segments is the peak area corresponding to the peptide segment as set forth in SEQ ID NO:8 plus the peak area corresponding to the peptide segment as set forth in SEQ ID NO:9. In some embodiments, the native peptide segment is a peptide segment as set forth in SEQ ID NO:6.

In some embodiments, the content of the truncated variant (e.g., as set forth in SEQ ID NO:5) in the composition comprising the recombinant human hyaluronidase and truncated variants thereof disclosed herein is about 0.01% to about 10% of the total amount of the recombinant human hyaluronidase and variants thereof, or any interval or value within the above ranges.

In some embodiments, the content of the oxidatively modified variant (e.g., the oxidatively modified variant resulting from oxidation of one of positions $M^{310}$ and $M^{313}$ in the sequence as set forth in any one of SEQ ID NOs: 1-4) in the composition comprising the recombinant human hyaluronidase and oxidatively modified variants thereof disclosed herein is about 0.01% to about 15% of the total amount of the recombinant human hyaluronidase and variant thereof, or any interval or value within the above ranges.

In this specification and in the claims, the term "about" refers to a range of values considered by one of ordinary skill in the art to be equivalent to the recited values (e.g., having the same function or result), e.g., +/-10% of the recited values. For example, with respect to the limitation that the content of the truncated variant is less than or equal to about 10% of the total amount of the recombinant human hyaluronidase and variants thereof, it will be understood by those skilled in the art that if the content of the truncated variant is 11%, it is also within the range claimed in the present application.

In a specific embodiment, the content of the above truncated variant is 3%-11%, for example 3.26%-10.96%, of the total amount of the recombinant human hyaluronidase and variants thereof. In another specific embodiment, the content of the oxidatively modified variant is 5%-16%, for example 5.31%-15.42%, of the total amount of the recombinant human hyaluronidase and variants thereof.

Also disclosed herein is a pharmaceutical formulation for preparing the composition disclosed herein, i.e., the composition is mixed with an optional pharmaceutically acceptable carrier, and stored in a lyophilized formulation or aqueous solution. The pharmaceutically acceptable carrier is non-toxic to the recipient at the dosages and concentrations employed. In some embodiments, the pharmaceutically acceptable carrier comprises water; buffering agents such as phosphates, citrates and other organic acids; antioxidants such as ascorbic acid and methionine; preservatives; hydrophilic polymers such as polyvinylpyrrolidone; chelating agents such as EDTA and the like. In some embodiments, the formulations for *in vivo* administration must be sterile. This can easily be achieved by filtration using a sterile filter membrane.

In this specification and in the claims, the phrases "include", "comprise" and "contain" mean "include but are not limited to", and do not intend to exclude other parts, additives, components or steps.

It should be understood that features, characteristics, components, or steps described in a particular aspect, embodiment, or example of the present application can be applied to any other aspect, embodiment, or example described herein unless contradicted thereby.

The above disclosure generally describes the present invention, which will be further exemplified by the following Examples. These examples are described merely to illustrate the invention and are not intended to limit the scope of the invention. Although specific terms and values are used herein, such terms and values are also understood to be exemplary and do not limit the scope of the invention. Unless otherwise specified, the experimental methods and techniques in this specification are those well known to a person skilled in the art.

**EXAMPLES**

**Example 1. Construction and Screening of Cell Lines Expressing Recombinant Human Hyaluronidase**

A nucleic acid sequence (the amino acid sequence of which was set forth in SEQ ID NO: 1) encoding the recombinant human hyaluronidase of the present disclosure was synthesized by a gene synthesis company, and was ligated into the polyclonal insertion site of an expression vector. After completion of construction, the plasmid was extracted after the sequence was verified to be correct by sequencing.

The recombinant expression plasmid was introduced into CHOZN® GS$^{-/-}$ blank cells by electrotransfection, and the transfected cells were inoculated into a 96-well plate at a density of 3,000-10,000 cells/well. After the inoculation was completed, the 96-well plate was placed in a carbon dioxide incubator for static culture under the culture conditions of 36.5°C and 5% $CO_2$. After the cells reached confluence in the wells, the expression levels were evaluated by dot blot techniques (Dot-blot), and cell pools with higher expression levels were selected and expanded into a 24-well plate. Cell pools with high expression levels were screened for fed-batch culture in shaker flasks by stepwise scale-up cultivation and screening in 96-well plates, 24-well plates, shaker tubes, shaker flasks, etc., and high-yield cell pools were screened for monoclonal cell line screening based on the combined results of cell number and yield.

The cell pools with higher expression levels were inoculated into a 96-well plate at an average density of 0.5 cells/well using a limited dilution method. After the inoculation was completed, the 96-well plate was placed in a carbon dioxide incubator for static culture under the culture conditions of 36.5°C, 5% $CO_2$, and 80% humidity. After the cells reached confluence in the wells, high-yield monoclonal cell lines were finally selected for fed-batch culture in shaker flasks by stepwise scale-up cultivation and screening in 96-well plates, 24-well plates, shaker tubes, shaker flasks, etc. A monoclonal cell line expressing recombinant human hyaluronidase was finally screened and obtained based on the combined results of cell growth, expression levels, product quality, and the like.

## Example 2. Preparation of Recombinant Human Hyaluronidase Using Cells from Cell Pools

In this example, a fed-batch culture was carried out in a bioreactor. The cells in the cell pool prepared in Example 1 were thawed in a 37°C water-bath pan, and resuscitated and cultured in a basal medium, and subcultured and expanded once every 2-4 days. After expansion to an appropriate volume in the basal medium, the cells were inoculated into a bioreactor at a density of $0.3 \times 10^6$-$1 \times 10^6$ cells/ml, and cultured at a temperature of $36.5 \pm 1$°C and a pH value of 6.8-7.2 under stirring at a rate of 250 rpm. Intermittent feeding was performed from day $4 \pm 1$, the feeding amount was 3-7% of the initial culture volume. On day $6 \pm 2$ of the culture period, the culture temperature was adjusted to $32.5 \pm 1$°C and maintained until the end of the culture period. Samples were taken at regular intervals during the culture, and the cells were counted using CountStar. The density of viable cells and changes in cell viability in the cell broth were monitored, and the content of the recombinant human hyaluronidase in the cell culture solution was determined by RP-HPLC. The enzymatic activity of recombinant human hyaluronidase in cell culture solution was detected by the turbidimetric method.

The cell culture solution was harvested, and was subjected to deep filtration and sterilization filtration to obtain clarified cell culture solution. The clarified cell culture solution was purified, and the purification steps include anion exchange chromatography, affinity chromatography, hydrophobic chromatography, etc. to prepare a stock solution.

## Example 3. Production of Recombinant Human Hyaluronidase Using Monoclonal Cell Lines

In this example, a fed-batch culture was carried out in a bioreactor. The monoclonal cell lines prepared in Example 1 were thawed in a 37°C water-bath pan, and resuscitated and cultured in a basal medium, and subcultured and expanded once every 2-4 days. After expansion to an appropriate volume in the basal medium, the cells were inoculated into a bioreactor at a density of $0.3 \times 10^6$-$1 \times 10^6$ cells/ml, and cultured at a temperature of $36.5 \pm 1$°C and a pH value of 6.8-7.2 under stirring at a rate of 250 rpm. Intermittent feeding was performed from days 2-4, the feeding amount was 3-7% of the initial culture volume. On day $6 \pm 2$ of the culture period, the culture temperature was adjusted to $32.5 \pm 1$°C and maintained until the end of the culture period. Samples were taken at regular intervals during the culture, and the cells were counted using CountStar. The density of viable cells and changes in cell viability in the cell culture solution were monitored, and the content of the recombinant human hyaluronidase in the cell culture solution was determined by RP-HPLC. The enzymatic activity of recombinant human hyaluronidase in cell culture broth was detected by the turbidimetric method.

The cell culture solution was harvested, and was subjected to deep filtration and sterilization filtration to obtain clarified cell culture solution. The clarified cell culture solution was purified, and the purification steps include anion exchange chromatography, affinity chromatography, hydrophobic chromatography, etc. to prepare a stock solution.

## Example 4. Analysis of Truncated Variants Generated by Cleavage at $R^{311}/S^{312}$ of Recombinant Human Hyaluronidase

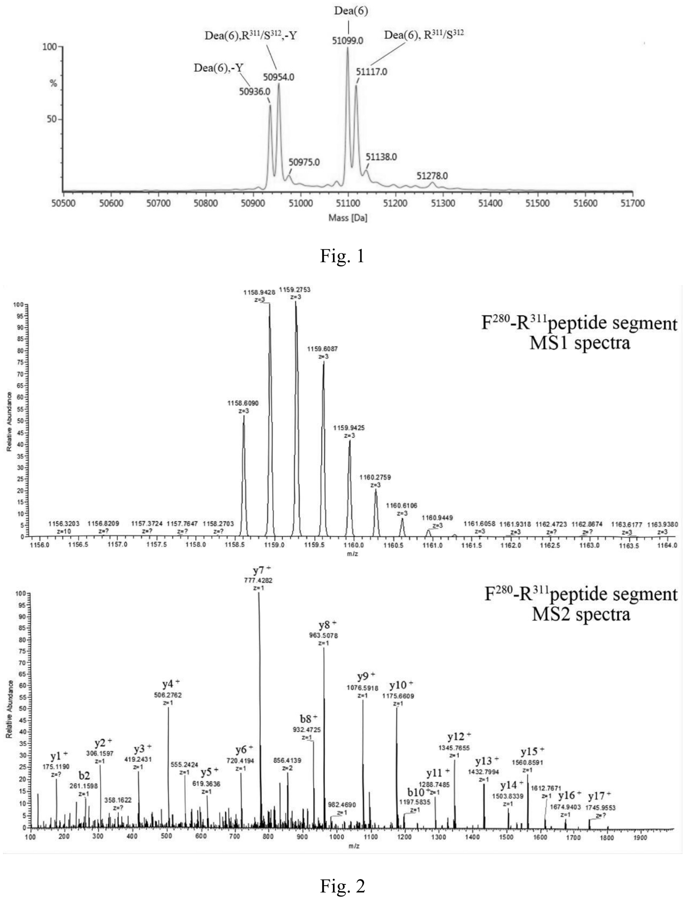

An analysis of N-glycan-cleaved non-reduced intact molecular weight was conducted on the recombinant human hyaluronidase sample prepared in Example 2. As the sample was treated with PNGase F, cleavage of N-glycans resulted in the conversion of glycosylated asparagine (N) to aspartic acid (D), i.e., deamidation modification (Dea). In addition, the C-terminus of hyaluronidase was susceptible to C-terminal truncation (-Y). Consequently, in the analysis of N-glycan-cleaved non-reduced intact molecular weight, the Dea(6) modified component and the Dea(6) and -Y modified component can be observed. In addition to the two components, corresponding components with a 18 Da molecular weight increase were found, namely Dea (6) and $R^{311}/S^{312}$ cleavage modified component as well as Dea (6), -Y, and $R^{311}/S^{312}$ cleavage modified component. When hyaluronidase undergoes cleavage at the $R^{311}/S^{312}$ site, the two resulting fragments remain connected via the $C^{25}$-$C^{320}$ disulfide bond, as indicated by the disulfide linkage pattern in SEQ ID NO:5, and therefore the molecular weight was about 18Da larger compared to the components without $R^{311}/S^{312}$ cleavage. This finding is consistent with the data obtained from the N-glycan-cleaved, non-reduced intact molecular weight analysis (see FIG. 1).

To further investigate this modification, the sample was digested with Lysine endopeptidase (Lys-C) and analyzed by LC-MS/MS. While digestion of the recombinant human hyaluronidase sample with Lys-C is expected to yield a peptide segment spanning $F^{280}$-$K^{314}$, and theoretically it would not produce $F^{280}$-$R^{311}$ peptide segment. However, a distinct signal corresponding to the $F^{280}$-$R^{311}$ peptide was detected in the sample. The identity of this $F^{280}$-$R^{311}$ peptide was confirmed by both MS1 and MS2 spectra (see FIG. 2).

The recombinant human hyaluronidase sample should contain impurities with $R^{311}/S^{312}$ cleavage modification based on the results of N-glycan-cleaved non-reduced intact molecular weight analysis and peptide mapping analysis.

Urokinase, also known as urokinase-type plasminogen activator (uPA), is a serine protease encoded by the PLAU gene. Urokinase was initially isolated from urine and is also present in blood and the extracellular matrix of many tissues. Urokinase has been reported to have good enzymatic activity for SGR/X motif, and the enzymatic activity is higher when X is S (the sequence of enzymatic activity corresponding to X is S >R, K, A). During the detection and analysis of recombinant human hyaluronidase samples, variants (one of the possible sequences of which is set forth in SEQ ID NO:5) produced by truncation at position $R^{311}/S^{312}$ were found. This truncation site mimics the enzymatically cleavable recognition sequence of urokinase-type plasminogen activator (uPA). Consequently, we employed uPA to achieve site-specific proteolysis at this location. By optimizing the reaction conditions, position $R^{311}/S^{312}$ was digested by uPA, which can be used for the preparation of the samples truncated at this position.

Variants by cleavage at $R^{311}/S^{312}$ (one of the exemplary sequences of which was set forth in SEQ ID NO:5) were prepared by digesting the recombinant human hyaluronidase sample prepared in Example 3 with uPA.The preparation process was as follows. In a PBS buffer system at pH 7.4, uPA was added in the ratio of protein to uPA (m/m) of 20:1, 10:1 and 5:1, respectively, and the sample without uPA was used as a control. After mixing well, all samples were incubated overnight at 37°C to prepare samples with different ratios of $R^{311}/S^{312}$ truncated variants.

Calculation of the truncation ratio of the $R^{311}/S^{312}$ truncated variant samples was as follows. Different ratios of the $R^{311}/S^{312}$ truncated variant samples obtained by using uPA were added with Lys-C at a ratio of protein: Lys-C (m/v) of 20:1 in a 100 mM Tris-HCl buffer system at pH 7.4, and were digested with Lys-C, mixed well and then were incubated at 37°C for 6 h, after which the obtained digested samples were subjected to LC-MS/MS analysis. After the detection was completed, the information about the truncated modified peptide segment and the non-truncated modified peptide segment, such as the mass-to-charge ratio and retention time, was determined by using the mass spectrometric data obtained by the data processing software. XIC chromatograms of the truncated modified peptide segment (hereinafter the "truncated peptide segment", the sequence of which was set forth in SEQ ID NO:7) and the non-truncated modified peptide segment (hereinafter the "native peptide segment", the sequence of which was set forth in SEQ ID NO:6) were respectively extracted from the mass spectrometric data of the samples, and were integrated to obtain peak areas of each component. The contents of the truncated variants generated by cleavage at $R^{311}/S^{312}$ in each sample were calculated according to the following formula:

The proportion of truncated modified variant = XIC area of truncated modified peptide segment/(XIC area of truncated modified peptide segment + XIC area of native peptide segment) $\times$ 100%.

The recombinant human hyaluronidase samples with different $R^{311}/S^{312}$ truncation ratios were obtained by addition of different proportions of uPA for digestion. The samples were digested with Lys-C, and the proportions of truncated variants generated by cleavage at $R^{311}/S^{312}$ can be calculated by the peak areas of the peptide segments. The proportions of truncated variants in each sample were shown in the following table (Table 1).

Table 1. Results of truncated modification proportion of $R^{311}/S^{312}$ in samples digested by uPA with different ratios

| Truncated peptide segment | Sample name | Addition ratio of uPA (m/m) | Truncated variant proportion (%) |
|---|---|---|---|
| FLSQDELVYTFGETVA LGASGIVIWGTLSIMR $^{311}$(its corresponding native peptide segment was FLSQDELVYTFGETVA LGASGIVIWGTLSIMR $^{311}/S^{312}$MK) | Control sample | No uPA | 3.26 |
| | Sample 1 | 20:1 | 4.04 |
| | Sample 2 | 10:1 | 10.96 |
| | Sample 3 | 5:1 | 42.65 |

The activities of the above recombinant human hyaluronidase samples containing different proportions of truncated variants were determined. The activities of these products were determined by the turbidimetric method in accordance with General Chapter 1207 "Hyaluronidase Assay" in Volume IV of the Pharmacopoeia of the People's Republic of China (2020 Edition). The principle is that a hyaluronic acid solution can form a stable colloidal solution with acidified serum to produce turbidity, and the hyaluronidase can enzymatically hydrolyze the substrate hyaluronic acid, thereby reducing the turbidity of the solution. A standard curve was plotted by determining different concentrations of standards and their corresponding absorbance, and the activities of these products were thus determined.

The reference of these products (calibrated by the national standard of hyaluronidase) was used as a standard. The standard was diluted in series to plot a standard curve. The activities of the samples after digestion of the products were determined according to the standard curve. The specific manipulations were as follows.

The reference was diluted to 40, 32, 24, 26, 8, 4, and 0 IU/ml as standard curve points. Meanwhile, the digested samples were respectively diluted 5000-fold and 10000-fold as test samples. The test samples and standard and the reaction plate containing the potassium hyaluronate solution were preheated at 37°C for 10 min, and the test samples and standard were mixed with the potassium hyaluronate working solution, and the standard and test samples enzymatically hydrolyzed the potassium hyaluronate solution at 37°C. After incubation of 6~8min at 37°C, the reaction was terminated by addition of acidified serum solution, and finally solutions of different turbidities were obtained. A standard curve (see FIG. 1) was plotted by measuring standards with different activities and their absorbance at 640nm, so that the activity values of the test samples were calculated from the absorbance thereof.

Table 2. Absorbance data at each point in standard curve

| Sample | Concentration Units/ml | BackCalcConc | Wells | Value | MeanValue | SD | CV |
|---|---|---|---|---|---|---|---|
| 01 | 40.000 | 40.165 | A1 | 0.079 | 0.084 | 0.005 | 6.4 |
| | | 39.695 | A2 | 0.083 | | | |
| | | 38.886 | A3 | 0.090 | | | |
| 02 | 32.000 | 33.214 | B1 | 0.137 | 0.135 | 0.008 | 5.8 |
| | | 34.469 | B2 | 0.127 | | | |
| | | 32.623 | B3 | 0.142 | | | |
| 03 | 24.000 | 25.648 | C1 | 0.200 | 0.208 | 0.009 | 4.2 |
| | | 24.695 | C2 | 0.207 | | | |
| | | 23.536 | C3 | 0.217 | | | |
| 04 | 16.000 | 14.969 | D1 | 0.288 | 0.294 | 0.009 | 3.0 |
| | | 14.739 | D2 | 0.290 | | | |
| | | 13.014 | D3 | 0.304 | | | |
| 05 | 8.000 | 6.606 | E1 | 0.357 | 0.359 | 0.002 | 0.7 |
| | | 6.546 | E2 | 0.358 | | | |
| | | 6.075 | E3 | 0.362 | | | |
| 06 | 4.000 | 2.950 | F1 | 0.388 | 0.386 | 0.004 | 0.9 |
| | | 3.650 | F2 | 0.382 | | | |
| | | 2.865 | F3 | 0.388 | | | |
| 07 | 0.000 | 2.431 | G1 | 0.392 | 0.391 | 0.001 | 0.2 |
| | | 2.636 | G2 | 0.390 | | | |
| | | 2.588 | G3 | 0.391 | | | |

The absorbance values of the test samples were substituted into the standard curve in FIG. 1. Upon calculation, the activity results of the digested samples were as shown in Table 3.

Table 3

| Sample name | Proportion of truncated variant (%) | Result of activity test (IU/ml) |
|---|---|---|
| Control sample | 3.26 | 115040 |
| Sample 1 | 4.04 | 90093 |
| Sample 2 | 10.96 | 88300 |
| Sample 3 | 42.65 | <LOQ* |
| *: Detection value below the quantitative limit of this method. | | |

The results showed that the content of the truncated variants produced by cleavage at $R^{311}/S^{312}$ in the

recombinant human hyaluronidase can affect the activity of the recombinant human hyaluronidase samples. The activity of the recombinant human hyaluronidase samples was gradually reduced as the content of the above truncated variants was gradually increased. Therefore, it is necessary to control the content of the above truncated variants in the recombinant human hyaluronidase-containing products.

In order to ensure the activity of the recombinant human hyaluronidase-containing products and make the truncated variants not significantly affect the enzymatic activity (i.e., not less than 70% of the initial activity), it is necessary to control the proportion or content of the truncated variants, e.g., not more than about 10% of the total amount of the recombinant human hyaluronidase and variants thereof.

**Example 5. Analysis of Oxidatively Modified Variants Produced by Oxidation at $M^{310}$ or $M^{313}$ of Recombinant Human Hyaluronidase**

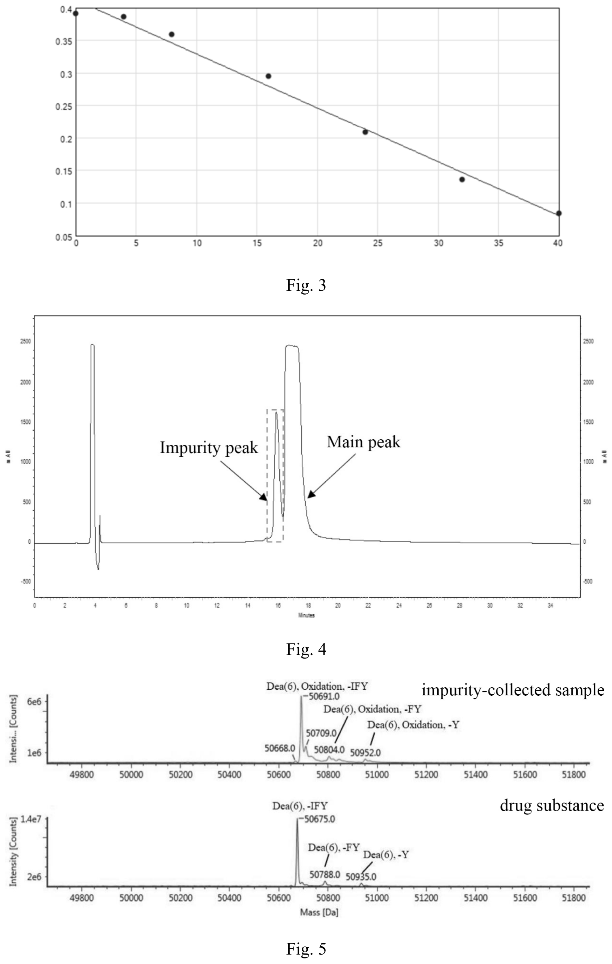

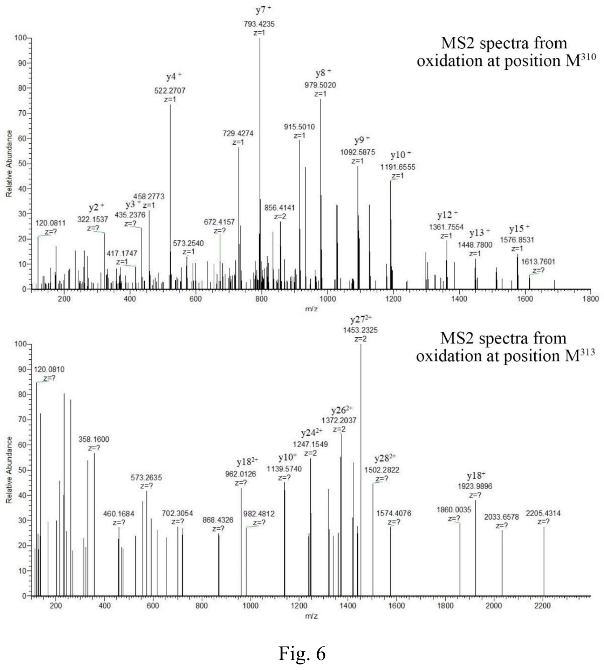

When the recombinant human hyaluronidase sample prepared in Example 3 was analyzed by RP-HPLC, an impurity peak was found in front of the main peak (see FIG. 4). The impurity peak was separated and collected, and the collected impurity peak and the stock solution sample were subjected to the N-glycan-cleaved non-reduced intact molecular weight analysis (see FIG. 5). The results showed that the main component of the stock solution sample was the Dea (6) and -IFY modified component, and the molecular weight (50691Da) of the main component of the impurity peak sample was about 16Da higher than that (50675Da) of the main component of the stock solution sample, which should be caused by oxidative modification of the recombinant human hyaluronidase, resulting in the Dea(6), oxidation, and -IFY modified components. The peptide mapping of the sample collected from the impurity peak and the stock solution sample was then analyzed for further confirmation (see FIG. 6). The results showed that an obviously oxidized modification was occurred at position $M^{310}$ or $M^{313}$ in the impurity peak sample compared to the stock solution sample, further confirming that the impurity peak component was mainly caused by the oxidation at position $M^{310}$ or $M^{313}$. The result of the peptide mapping data analysis showed that the percentage of oxidative modification at position $M^{310}$ or $M^{313}$ in the recombinant human hyaluronidase sample was about 5% of the total amount of the recombinant human hyaluronidase and variants thereof.

The main peak and the impurity peak in front of the main peak of the recombinant human hyaluronidase sample prepared in Example 3 were synchronously collected by using the Agilent 1260 liquid chromatography automatic fraction collector module. The injection volume of each needle was 500 μg, with a total of 27 injections. The components of the main peak and impurity peak collected from each injection were separately taken out and placed in the corresponding centrifuge tubes, with approximately 300μl/tube. The components were concentrated and evaporated by a centrifugal concentrator for a suitable time (about 5 hours), until the liquid in the centrifugal tube was completely evaporated to dryness. 150 μl of auxiliary buffer solutions of the stock solution were taken and added to the bottom of the centrifuge tubes, respectively, so as to sufficiently dissolve the protein in the centrifuge tubes. An aliquot of 20 μl was taken from each tube for localization and purity analysis of the collected components. A typical chromatogram of the collected components was shown in Figure 2. The result of determining protein concentration in the component collected by RP-HPLC was shown in Table 4.

Table 4. Result of determining protein concentration in the component collected by RP-HPLC

| Sample name | Protein content (mg/ml) |
|---|---|
| Collected impurity component | 1.3 |

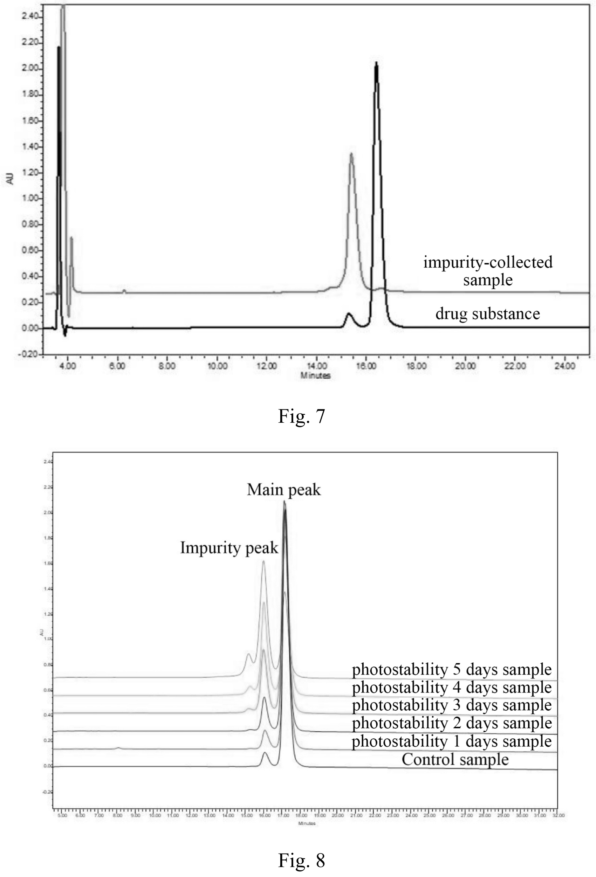

The protein contents of the collected components were determined using a Lunatic micro spectrometer, and then the collected components were subjected to RP-HPLC for confirming localization and purity analysis using a Waters e2695 liquid chromatograph. The chromatogram for confirming localization was shown in Figure 7 and the results of purity analysis were shown in Table 5.

Table 5: Results of purity analysis of the collected components by RP-HPLC

| Sample name | Total impurities in front of main peak (%) | Main peak (%) |
|---|---|---|
| Collected impurity component | 96.4 | 3.6 |
| Stock solution before purification | 5.3 | 94.7 |

The impurity peak and the main peak obtained by collecting the liquid phases, as well as the stock solution before purification were added with trypsin in a ratio of protein: trypsin (m/v) of 10: 1 in a 50mM $NH_4HCO_3$ buffer system, and were digested with trypsin, mixed well, and then incubated at 37°C for 60 minutes. After which, the obtained digested samples were subjected to LC-MS/MS analysis. After the detection was completed, the information about the $M^{310}$ or $M^{313}$ oxidatively modified peptide segments and the native peptide segment, such as the mass-to-charge ratio and retention time, was determined by using the mass spectrometric data obtained by the data processing software. XIC chromatograms of the oxidatively modified peptide segments and the native peptide segment were respectively extracted from the mass spectrometric data of the samples, and were integrated to obtain peak areas of each component. The contents of the $M^{310}$ or $M^{313}$ oxidatively modified variants in each sample were calculated according to the following formula:

The proportion of oxidatively modified variants = XIC area of oxidatively modified peptide segments /(XIC area of oxidatively modified peptide segments + XIC area of native peptide segment) × 100%.

When calculating the proportion of oxidative modification at position $M^{310}$, the sequence of the native peptide fragment was FLSQDELVYTFGETVALGASGIVIWGTLSIMR$^{311}$ (as set forth in SEQ ID NO:7). When calculating the proportion of oxidative modification at position $M^{313}$, as the length of the peptide segment SM$^{313}$K where position $M^{313}$ is located is short due to trypsin enzyme, the chromatographic column cannot retain it. Therefore, when calculating the proportion of oxidative modification at position $M^{313}$, the proportion of oxidative modification was calculated by using the native peptide segment, the sequence of which was FLSQDELVYTFGETVALGASGIVIWGTLSIMR$^{311}$S$^{312}$MK (as set forth in SEQ ID NO:6).

The sequence of the oxidatively modified peptide segment at position $M^{310}$ was FLSQDELVYTFGETVALGAS-GIVIWGTLSIM$^{310}$(Oxi)R (SEQ ID NO:8), and the sequence of the oxidatively modified peptide segment at position $M^{313}$ was FLSQDELVYTFGETVALGASGIVIWGTLSIMRSM$^{313}$(Oxi)K(SEQ ID NO:9).

The XIC area of the above oxidatively modified peptide segments was the peak area corresponding to the peptide segment as set forth in SEQ ID NO:8 plus the peak area corresponding to the peptide segment as set forth in SEQ ID NO:9.

Post-translational modification analysis was conducted on the impurity in front of the main peak collected by reversed phase chromatography and the stock sample. The results showed that the oxidation ratio at position $M^{310}$ or $M^{313}$ in the impurity was significantly higher than that in the stock solution sample, indicating that the impurity peak in front of the main peak was mainly caused by oxidation at position $M^{310}$ or $M^{313}$. The oxidation ratios at position $M^{310}$ or $M^{313}$ for each sample were shown in the table below (Table 6).

Table 6: Oxidation results at position $M^{310}$ or $M^{313}$ in the component collected by RP-HPLC and stock solution

| Modification type | Modification position | Modification ratio (%) | |
|---|---|---|---|
| | | Impurity peak | Stock solution |
| | $M^{310}$ | 18.66 | 1.47 |
| | $M^{313}$ | 92.35 | 3.07 |

The stock solution sample was examined using a stability chamber at a temperature of 25°C under light exposure of 5000lux for 1 to 5 days. Then, RP-HPLC purity analysis was performed on the light-exposed stability test samples and the control samples (unexposed samples) using a Waters e2695 liquid chromatograph. A typical chromatogram was shown in Figure 4.

The light-exposed stability test sample and the control sample were added with Lys-C in a ratio of protein: Lys-C (m/v) of 10: 1 in a 100mM Tris-HCl buffer system at pH 7.4, and were digested with Lys-C, mixed well, and then incubated at 37°C for 60 minutes, after which the obtained digested samples were subjected to LC-MS/MS analysis. After the detection was completed, the acquired mass spectrometric data was used to calculate the oxidation ratio at position $M^{310}$ or $M^{313}$ according to the above calculation method.

The result showed that the oxidation ratio at position $M^{310}$ or $M^{313}$ in the light-exposed stability test sample was significantly higher than that in the control sample, and the sum of the oxidation ratios at position $M^{310}$ or $M^{313}$ in each sample was shown in the following table (Table 7).

Table 7: Result of oxidative modification at position $M^{310}$ or $M^{313}$ in the light-exposed stability test sample and the control sample

| Modification type | Modification position | Modification ratio (%) | | | | | |
|---|---|---|---|---|---|---|---|
| | | Control Sample | 1-day light-exposed sample | 2-day light-exposed sample | 3-day light-exposed sample | 4-day light-exposed sample | 5-day light-exposed sample |
| Oxidation | $M^{310}$ or $M^{313}$ | 5.31 | 7.58 | 15.42 | 36.18 | 57.83 | 90.10 |

The activities of the light-exposed samples and light-protected sample (control sample) of the recombinant human hyaluronidase were determined, and the determination method was as described in Example 3.

After calculation, the activity results of the light-exposed samples and the control sample were shown in Table 8 below.

Table 8

| Sample name | Detection Result (IU/ml) |
|---|---|
| Control sample | 1192800 |
| 1-day light-exposed sample | 1018700 |
| 2-day light-exposed sample | 865280 |
| 3-day light-exposed sample | 546840 |
| 4-day light-exposed sample | 345860 |
| 5-day light-exposed sample | 56948 |

The results showed that the impurity peak in front of the main peak as can be seen by RP-HPLC analysis was caused by oxidation modification at position $M^{310}$ or $M^{313}$, and the oxidatively modified variants produced by oxidation at position $M^{310}$ or $M^{313}$ can affect the activity of the product. The activity of the recombinant human hyaluronidase sample gradually decreased as the content of the oxidatively modified variants gradually increased.

In order to ensure the quality of the recombinant human hyaluronidase-containing product, the activity of the recombinant human hyaluronidase should not be lower than 80,000 IU/ml. Therefore, it is necessary to control the proportion or content of the above-mentioned oxidation-modified variants, for example, to control them to be not higher than about 15% of the total amount of recombinant human hyaluronidase and variants thereof.

Various changes and equivalent substitutions can be made to the embodiments disclosed herein without departing from the spirit and scope of the disclosure. Unless otherwise indicated in the context, any feature, step, or embodiment of the disclosed embodiments in the present application can be used in combination with any other features or embodiments.

**Claims**

1. A composition comprising recombinant human hyaluronidase and variants thereof, wherein the variants are an oxidatively modified variant resulting from oxidation at position $M^{310}$ and an oxidatively modified variant resulting from oxidation at position $M^{313}$ of the recombinant human hyaluronidase, wherein the sum of the contents of the two oxidatively modified variants is less than or equal to about 15% of the total amount of the recombinant human hyaluronidase and variants thereof in the composition, calculated based on a Lys-C digestion peptide mass fingerprinting analysis; and
wherein the positions are numbered with reference to the sequence as set forth in SEQ ID NO:1.

2. The composition of claim 1, wherein the content of the recombinant human hyaluronidase is greater than or equal to about 85% of the total amount of the recombinant human hyaluronidase and variants thereof in the composition, calculated based on the Lys-C digestion peptide mass fingerprinting analysis.

3. The composition of claim 1 or 2, wherein the sum of the contents of the two oxidatively modified variants is greater than or equal to about 0.01%, greater than or equal to about 0.05%, greater than or equal to about 0.1%, greater than or equal to about 0.5%, greater than or equal to about 1%, greater than or equal to about 1.5%, greater than or equal to about 2%, greater than or equal to about 2.5%, greater than or equal to about 3%, greater than or equal to about 3.5%, greater than or equal to about 4%, greater than or equal to about 4.5%, or greater than or equal to about 5% of the total amount of the recombinant human hyaluronidase and variants thereof in the composition, calculated based on the Lys-C digestion peptide mass fingerprinting analysis.

4. The composition of any one of claims 1-3, further comprising a truncated variant resulting from a cleavage between positions $R^{311}$ and $S^{312}$ of the recombinant human hyaluronidase, wherein the content of the truncated variant is less than or equal to about 10% of the total amount of the recombinant human hyaluronidase and variants thereof in the composition, calculated based on the Lys-C digestion peptide mass fingerprinting analysis; and wherein the positions are numbered with reference to the sequence as set forth in SEQ ID NO:1.

5. The composition of claim 4, wherein the content of the truncated variant is greater than or equal to about 0.01%, greater than or equal to about 0.05%, greater than or equal to about 0.1%, greater than or equal to about 0.5%, greater than or equal to about 1%, greater than or equal to about 1.5%, greater than or equal to about 2%, greater than or equal to about 2.5%, or greater than or equal to about 3% of the total amount of the recombinant human hyaluronidase and variants thereof in the composition, calculated based on the Lys-C digestion peptide mass fingerprinting analysis.

6. A composition comprising recombinant human hyaluronidase and a variant thereof, wherein the variant is a truncated variant resulting from a cleavage between positions $R^{311}$ and $S^{312}$ of the recombinant human hyaluronidase, and wherein the content of the truncated variant is less than or equal to about 10% of the total amount of the recombinant human hyaluronidase and the variant thereof in the composition, calculated based on a Lys-C digestion peptide mass fingerprinting analysis; and wherein the positions are numbered with reference to the sequence as set forth in SEQ ID NO:1.

7. The composition of claim 6, wherein the content of the recombinant human hyaluronidase is greater than or equal to about 90% of the total amount of the recombinant human hyaluronidase and the variant thereof in the composition, calculated based on the Lys-C digestion peptide mass fingerprinting analysis.

8. The composition of claim 6 or 7, wherein the content of the truncated variant is greater than or equal to about 0.01%, greater than or equal to about 0.05%, greater than or equal to about 0.1%, greater than or equal to about 0.5%, greater than or equal to about 1%, greater than or equal to about 1.5%, greater than or equal to about 2%, greater than or equal to about 2.5%, or greater than or equal to about 3% of the total amount of the recombinant human hyaluronidase and the variant thereof in the composition, calculated based on the Lys-C digestion peptide mass fingerprinting analysis.

9. A pharmaceutical formulation comprising the composition of any one of claims 1-5, and/or the composition of any one of claims 6-8, and one or more pharmaceutically acceptable carriers.

10. The pharmaceutical formulation of claim 9, further comprising an additional pharmaceutically active ingredient, preferably selected from the group consisting of immunoglobulins, recombinant proteins, synthetic polypeptides, RNA, DNA and chemical drugs.

11. The composition of any one of claims 1-5, or the pharmaceutical formulation of claim 9, wherein the Lys-C digestion peptide mass fingerprinting analysis comprises the following steps:

digesting the composition or pharmaceutical formulation with Lys-C followed by peptide mass fingerprinting analysis using LC-MS/MS;
extracting the extracted ion chromatograms (XICs) of two charge forms with the strongest responses of the oxidatively modified peptide segments and their corresponding native peptide segments after obtaining mass spectrometric data by LC-MS/MS analysis;
integrating to obtain respective peak area; and calculating the content of the oxidatively modified variants according to the following formula:

the proportion of oxidatively modified variants = (XIC area of oxidatively modified peptide segments)/(XIC area of oxidatively modified peptide segments + XIC area of native peptide segment) $\times$ 100%.

12. The composition of any one of claims 4-5 or 6-9, or the pharmaceutical formulation of claim 9, wherein the Lys-C digestion peptide mass fingerprinting analysis comprises the following:

digesting the composition or pharmaceutical formulation with Lys-C followed by peptide mass fingerprinting analysis using LC-MS/MS;
extracting the extracted ion chromatograms (XICs) of two charge forms which have the strongest responses of the truncated peptide segment and its corresponding native peptide segment after obtaining mass spectrometric data by LC-MS/MS analysis;
integrating to obtain respective peak area; and calculating the proportion of the truncated variant according to the following formula:

the proportion of truncated variant = (XIC area of truncated peptide segment)/(XIC area of truncated peptide segment + XIC area of native peptide segment) $\times$ 100%.

13. A method for quality inspection or quality control of a recombinant human hyaluronidase-containing product, comprising detecting the content of a recombinant human hyaluronidase variant in the product, wherein the variant is 1) a truncated variant resulting from a cleavage between positions $R^{311}$ and $S^{312}$ of the recombinant human hyaluronidase, or 2) an oxidatively modified variant resulting from oxidation at position $M^{310}$ and an oxidatively modified variant resulting from oxidation at position $M^{313}$ of the recombinant human hyaluronidase, wherein the positions are numbered with reference to the sequence as set forth in SEQ ID NO:1.

14. The method of claim 13, wherein the product meets medicinal requirements if the content of the truncated variant is detected to be less than or equal to about 10%; and/or
wherein the product meets medicinal requirements if the sum of the contents of the two oxidatively modified variants is detected to be less than or equal to about 15%.

15. Use of a recombinant human hyaluronidase variant in the quality inspection or quality control of a recombinant human hyaluronidase-containing product, wherein the recombinant human hyaluronidase variant is 1) a truncated variant resulting from a cleavage between positions $R^{311}$ and $S^{312}$ of the recombinant human hyaluronidase, or 2) an oxidatively modified variant resulting from oxidation at position $M^{310}$ and an oxidatively modified variant resulting from oxidation at position $M^{313}$ of the recombinant human hyaluronidase, wherein the positions are numbered with reference to the sequence as set forth in SEQ ID NO:1.

Fig. 1

Fig. 2

Fig. 3

Fig. 4

Fig. 5

Fig. 6

Fig. 7

Fig. 8

**REFERENCES CITED IN THE DESCRIPTION**

**Patent documents cited in the description**

- CN 202510827503
- CN 104244968 B
- CN 112203642 B


**Non-patent literature cited in the description**

- **GREGORY I FROST**. Recombinant human hyaluronidase (rHuPH20): an enabling platform for subcutaneous drug and fluid administration. *Expert Opinion on Drug Delivery*, 2007, vol. 4 (4), 427-440
- Hyaluronidase Assay. *Pharmacopoeia of the People's Republic of China*, 2020, vol. IV